# EUROPÄISCHE PATENTANMELDUNG

(11) **EP 2 428 245 A2**
(43) Veröffentlichungstag der Anmeldung: **14.03.2012**
(21) Anmeldenummer: 11180395.3
(22) Anmeldetag: 07.09.2011
(51) Int. Cl.: A61M 16/06

(54) **Beatmungsmaske, Beatmungsmaskenanordnung, Beatmungssystem und Verpackungseinheit**

(30) Priorität: 14.09.2010 DE 102010040771
(71) Anmelder: Med In Medical Innovations GmbH, 82178 Puchheim (DE)
(72) Erfinder: Koschany, Angelika, 81243 München (DE)
(74) Vertreter: Barth, Stephan Manuel

(57) **Zusammenfassung**

Beatmungsmaske (1) zum künstlichen Beatmen eines Patienten (29), mit: einem Nasenaufnahmeraum (2); und mindestens drei Befestigungslaschen (11, 12, 13) zum Befestigen der Beatmungsmaske (1) an dem Patienten. Die Erfindung betrifft ferner eine Beatmungsmaskenanordnung (27) mit einer derartigen Beatmungsmaske, einer auf dem Kopf (28) des Patienten angeordneten Mütze (30), und Befestigungsmitteln (24, 25, 26), zum Fixieren der Beatmungsmaske. Die Erfindung betrifft auch ein Beatmungssystem (34) mit einer derartigen Beatmungsmaskenanordnung und einer Beatmungsvorrichtung (35) zum Erzeugen eines kontinuierlichen positiven Atemwegdrucks. Zur Erfindung gehört auch eine Verpackungseinheit (42) mit einer derartigen Beatmungsmaske mit einem Saum (17) mit einer umlaufenden Nut (21); ein Dichtmittel (22), zur Abdichtung der Beatmungsmaske und einer Schutzfolie (23) zum Abdecken des Dichtmittels bis zum Gebrauch der Beatmungsmaske.

## Beschreibung

Die vorliegende Erfindung verweist hiermit auf die deutsche Gebrauchsmusterschrift DE 202 06 692 U1, deren gesamter Offenbarungsgehalt durch Querverweis miteinbezogen wird.

Die vorliegende Erfindung betrifft eine Beatmungsmaske zum künstlichen Beatmen eines Patienten, eine Beatmungsmaskenanordnung, ein Beatmungssystem und eine Verpackungseinheit. Die Beatmungsmaske ist insbesondere für Frühgeborene und Kleinkinder geeignet.

Beatmungsmasken werden unter anderem zur künstlichen Beatmung in Intensivstationen verwendet. Diese Beatmungsmasken sind in unterschiedlichen Größen und Formen für die typischen Nasenformen und Größen ausgebildet, so dass im Wesentlichen für jede Nasenform eine formschlüssige Beatmungsmaske verfügbar ist. Die Beatmungsmaske weist einen Anschlussbereich zum Anschließen an eine Beatmungspumpe auf. Die Beatmungsmaske weist ferner zwei Befestigungslaschen auf, welche jeweils an einer Seite der Beatmungsmaske angeordnet sind, welche entsprechend einem Nasenflügel der Nase des Patienten zugeordnet ist. Die Befestigungslaschen werden mittels eines Befestigungsmittels, wie beispielsweise eines Klettbandes, an dem Kopf des Patienten fixiert.

An der Nasenwurzel des Patienten kann die Beatmungsmaske jedoch leicht von dem Gesicht des Patienten abgehoben werden, beispielsweise bei einer Bewegung des Patienten. Hierdurch kann Beatmungsluft aus der Beatmungsmaske ausströmen. Hierdurch ist die Funktionalität der Beatmungsmaske nicht zuverlässig gewährleistet. Insbesondere kann bei einer Bewegung des Patienten eine Zugbelastung auf Verbindungsschläuche zwischen der Beatmungsmaske und der Beatmungspumpe aufgebracht werden, wodurch die Beatmungsmaske zumindest teilweise von dem Gesicht des Patienten abgehoben wird. Dies gilt es verständlicherweise zu vermeiden.

Hiervon ausgehend ist es Aufgabe der vorliegenden Erfindung, eine verbesserte Beatmungsmaske zur Verfügung zu stellen, welche die vorgenannten Nachteile beseitigt.

Diese Aufgabe wird gelöst durch eine Beatmungsmaske mit den Merkmalen des Patentanspruchs 1 und/oder durch eine Beatmungsmaskenanordnung mit den Merkmalen des Patentanspruchs 6 und/oder durch ein Beatmungssystem mit den Merkmalen des Patentanspruchs 9 und/oder durch eine Verpackungseinheit mit den Merkmalen des Patentanspruchs 12.

Demgemäß ist eine Beatmungsmaske zum künstlichen Beatmen eines Patienten vorgesehen, mit: einem Nasenaufnahmeraum; und mindestens drei Befestigungslaschen zum Befestigen der Beatmungsmaske an dem Patienten, wobei jeweils eine erste und zweite Befestigungslasche an einem jeweiligen Nasenflügelabschnitt des Nasenaufnahmeraumes und eine dritte Befestigungslasche an einem Nasenwurzelabschnitt des Nasenaufnahmeraumes angeordnet ist.

Ferner ist eine Beatmungsmaskenanordnung vorgesehen, mit: einer derartigen Beatmungsmaske; einer auf dem Kopf des Patienten angeordneten Mütze; und Befestigungsmitteln, welche zum Fixieren der Beatmungsmaske an dem Kopf des Patienten mit den Befestigungslaschen und mit der Mütze in Wirkverbindung sind.

Noch ferner ist ein Beatmungssystem vorgesehen, mit: einer derartigen Beatmungsmaskenanordnung; und einer Beatmungsvorrichtung zum Erzeugen eines kontinuierlichen positiven Atemwegdrucks.

Noch ferner ist eine Verpackungseinheit vorgesehen, mit: einer derartigen Beatmungsmaske, wobei die Beatmungsmaske einen Saum mit einer umlaufenden Nut aufweist; ein Dichtmittel, welches zur Abdichtung der Beatmungsmaske gegen das Gesicht des Patienten zumindest abschnittsweise in der umlaufenden Nut angeordnet ist; und einer Schutzfolie zum Abdecken des Dichtmittels bis zum Gebrauch der Beatmungsmaske.

Die Grundidee der vorliegenden Erfindung besteht darin, an dem Nasenwurzelabschnitt des Nasenaufnahmeraumes eine dritte Befestigungslasche vorzusehen, wodurch im Vergleich zu beschriebenen Lösungsansätzen ein sicherer Sitz der Beatmungsmaske auf dem Gesicht des Patienten stets gewährleistet ist. Hierdurch erhöhen sich die Zuverlässigkeit und der Tragekomfort der Beatmungsmaske signifikant.

Vorteilhafte Ausgestaltungen und Weiterbildungen sind in den Unteransprüchen und den Ausführungsformen angegeben.

Gemäß einer bevorzugten Ausführungsform der Beatmungsmaske sind eine erste Befestigungslasche und eine zweite Befestigungslasche auf einer gemeinsamen ersten Befestigungsachse angeordnet und eine dritte Befestigungslasche ist auf einer senkrecht zu der ersten Befestigungsachse ausgebildeten zweiten Befestigungsachse angeordnet. Mittels der senkrechten Anordnung der Befestigungsachsen zueinander ist ein sicherer Sitz der Beatmungsmaske auf dem Gesicht des Patienten gewährleistet, da die Beatmungsmaske an drei voneinander möglichst weit entfernten Punkten fixiert ist.

Gemäß einer weiter bevorzugten Ausführungsform der Beatmungsmaske weist diese einen umlaufenden Saum zur Auflage auf dem Gesicht des Patienten auf. Hierdurch wird die Auflagefläche der Beatmungsmaske auf dem Gesicht des Patienten vergrößert, wodurch sich der Tragekomfort für den Patienten nochmals erhöht und die Abdichtung zwischen der Beatmungsmaske und dem Gesicht des Patienten verbessert ist.

Gemäß einer weiter bevorzugten Weiterbildung der Beatmungsmaske weist der Saum eine umlaufende Nut zur Aufnahme eines Dichtmittels, insbesondere eines Silikongels, auf, wodurch die Abdichtung zwischen der Beatmungsmaske und dem Gesicht des Patienten weiter verbessert wird. Hierdurch erhöht sich die Zuverlässigkeit der Beatmungsmaske.

Gemäß einer weiter bevorzugten Ausführungsform der Beatmungsmaske sind die Befestigungslaschen an dem Saum angeordnet. Hierdurch ist die Beatmungsmaske besonders leicht herstellbar weil beispielsweise ein entsprechendes Formwerkzeug weniger komplex aufgebaut ist, wodurch sich die Kosten zur Herstellung der Beatmungsmaske reduzieren.

Gemäß einer bevorzugten Ausführungsform der Beatmungsmaskenanordnung sind die Befestigungsmittel als Klettbänder ausgebildet. Hierdurch ist eine besonders schnelle, variable und komfortable Fixierung der Beatmungsmaske an dem Patienten möglich, wodurch die Handhabung der Beatmungsmaske vereinfacht ist.

Gemäß einer weiter bevorzugten Ausführungsform der Beatmungsmaskenanordnung ist jeweils ein Befestigungsmittel mit einem jeweiligen Ohrabschnitt der Mütze und ein Befestigungsmittel mit einem Stirnabschnitt der Mütze in Wirkverbindung. Hierdurch wird die Beatmungsmaske sowohl in Richtung der Ohren des Patienten als auch in Richtung der Stirn des Patienten fixiert, wodurch ein Verrutschen der Beatmungsmaske unterbunden wird.

Gemäß einer bevorzugten Ausführungsform des Beatmungssystems weist die Mütze an einem Stirnabschnitt eine Befestigungseinrichtung zur Befestigung der Beatmungsvorrichtung an der Mütze auf. Hierdurch ist die Beatmungsvorrichtung an der Mütze festgelegt, wodurch ein Verrutschen der Beatmungsmaske durch eine Bewegung der Beatmungsvorrichtung, beispielsweise bei einem Bewegen des Patienten, zuverlässig verhindert wird.

Gemäß einer weiter bevorzugten Ausführungsform des Beatmungssystems ist die Befestigungseinrichtung als Kletteinrichtung ausgebildet, wodurch die Beatmungsvorrichtung besonders schnell und komfortabel an der Mütze fixierbar ist. Hierdurch wird die Handhabung des Beatmungssystems vereinfacht und beschleunigt.

Gemäß einer bevorzugten Ausführungsform der Verpackungseinheit ist das Dichtmittel als Silikongel ausgebildet. Hierdurch wird eine ausgezeichnete Verträglichkeit des Patienten gegenüber dem Dichtmittel erreicht, wodurch der Komfort des Patienten erhöht ist.

Die vorliegende Erfindung wird nachfolgend anhand bevorzugter Ausführungsformen und der beiliegenden Zeichnung erläutert. In der Zeichnung zeigen:
- Fig. 1: eine Rückansicht einer bevorzugten Ausführungsform einer Beatmungsmaske;
- Fig. 2: eine Schnittansicht der Beatmungsmaske gemäß der Schnittlinie II-II nach Fig. 1;
- Fig. 3: eine Vorderansicht der Beatmungsmaske gemäß Fig. 1 mit Befestigungsmitteln;
- Fig. 4: eine Vorderansicht einer bevorzugten Ausführungsform einer Beatmungsmaskenanordnung;
- Fig. 5: eine Vorderansicht einer bevorzugten Ausführungsform eines Beatmungssystems; und
- Fig. 6: eine weitere Vorderansicht des Beatmungssystems gemäß Fig. 5.

In den Figuren bezeichnen gleiche Bezugszeichen gleiche oder funktionsgleiche Elemente.

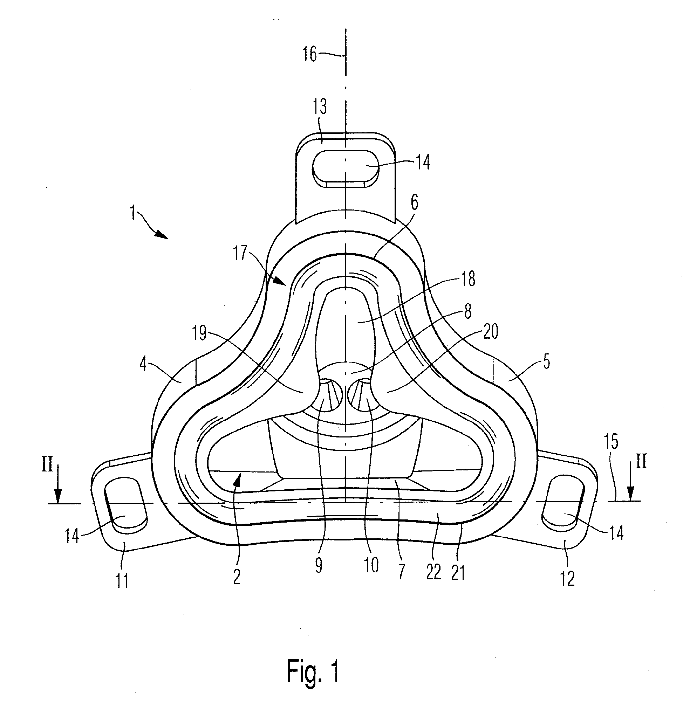

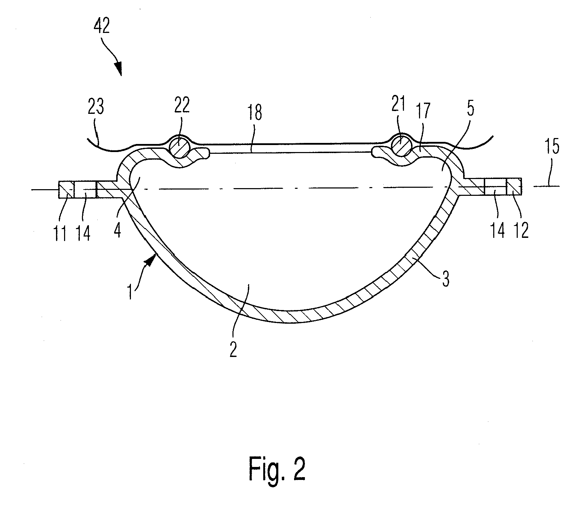

Die Fig. 1 und 2, auf die im Folgenden gleichzeitig Bezug genommen wird, illustrieren ein bevorzugtes Ausführungsbeispiel einer Beatmungsmaske 1 zum künstlichen Beatmen eines Patienten, insbesondere eines Säuglings oder Kleinkinds, in einer Rückansicht und in einer Schnittansicht.

Die Beatmungsmaske 1 weist vorzugsweise einen Nasenaufnahmeraum 2 zum zumindest abschnittsweisen Aufnehmen der Nase eines Patienten auf. Insbesondere ist der Nasenaufnahmeraum 2 an eine Geometrie der Nase des Patienten angepasst. Eine geometrische Form und ein Volumen des Nasenaufnahmeraums 2 ist vorzugsweise derart ausgebildet, dass bei einem Aufsetzen der Beatmungsmaske 1 auf das Gesicht des Patienten der Nasenaufnahmeraum 2 vorzugsweise vollständig oder nahezu vollständig von der Nase ausgefüllt ist, insbesondere umfasst die Beatmungsmaske 1 die Nase zumindest abschnittsweise formschlüssig. Eine Wandung 3 des Nasenaufnahmeraums 2 ist dabei vorzugsweise derart ausgebildet, dass diese nicht auf die Nase drückt. Die Beatmungsmaske 1 wird beispielsweise in unterschiedlichen Formen und Größen bereitgestellt. Vorzugsweise ist die Wandung 3 des Nasenaufnahmeraums 2 mit einem besonders weichen und nachgiebigen Material zur Anpassung an die Nase des Patienten gebildet. Insbesondere eignen sich als Materialen für die Wandung 3 weiche bzw. elastische Silikon- und/oder Polyurethankunststoffe. Die Beatmungsmaske 1 ist vorzugsweise als Einmalartikel ausgebildet.

Der Nasenaufnahmeraum 2 weist vorzugsweise zwei Nasenflügelabschnitte 4, 5, welche einem jeweiligen Nasenflügel der Nase des Patienten zugeordnet sind und einen Nasenwurzelabschnitt 6, welcher einer Nasenwurzel der Nase des Patienten zugeordnet ist, auf. Der Nasenaufnahmeraum 2 der Beatmungsmaske 2 weist weiterhin einen Nasenspitzenabschnitt 7, welcher einer Nasenspitze der Nase des Patienten zugeordnet ist, auf.

In dem Nasenspitzenabschnitt 7 des Nasenaufnahmeraums 2 ist vorzugsweise ein Anschlussstück 8 zum Anschließen einer Beatmungsvorrichtung an die Beatmungsmaske 1 vorgesehen. Die Beatmungsvorrichtung dient zum Erzeugen eines kontinuierlichen positiven Atemwegdrucks, das heißt, der Patient atmet gegen einen Überdruck. Eine derartige Beatmungsvorrichtung ist beispielsweise in der deutschen Gebrauchsmusterschrift DE 202 06 692 U1 beschrieben. Das Anschlussstück 8 weist vorzugsweise in etwa die Form eines Quaders auf, welcher beispielsweise zwei nebeneinander angeordnete Durchbrüche 9, 10 aufweist. Bevorzugt ist das Anschlussstück 8 einstückig mit der Wandung 3 des Nasenaufnahmeraumes 2 ausgebildet. Die Durchbrüche 9, 10 dienen zum Zuführen von Frischluft zu dem Patienten und zum Abführen von verbrauchter Atemluft des Patienten. Beispielsweise ist der Durchbruch 9 als Gaseinlass 9 und der Durchbruch 10 als Gasauslass 10 der Beatmungsmaske 1 ausgebildet. Mittels der Beatmungsvorrichtung wird der Nasenaufnahmeraum 2 kontinuierlich mit Gas durchspült und in der Ausatemluft des Patienten enthaltenes Kohlendioxid wird ausgewaschen.

Die Beatmungsmaske 1 weist ferner Befestigungslaschen 11, 12, 13 auf. Die Anzahl der Befestigungslaschen ist beliebig, vorzugsweise weist die Beatmungsmaske 1 jedoch mindestens drei Befestigungslaschen 11, 12, 13 auf. Jede Befestigungslasche 11, 12, 13 weist einen Durchbruch 14 zum jeweiligen Durchführen eines Befestigungsmittels auf. Der Durchbruch 14 ist beispielsweise in Form einer Ellipse, eines Kreises, eines verrundeten Rechtecks oder dergleichen ausgebildet. Vorzugsweise sind die Befestigungslaschen 11, 12, 13 einstückig mit der Wandung 3 des Nasenaufnahmeraumes 2 ausgebildet. Beispielsweise sind die Befestigungslaschen 11, 12, 13 bezüglich einer Auflagefläche 17 der Beatmungsmaske 1 etwas zurückgesetzt, wodurch sich ein verbesserter Andruck der Beatmungsmaske 1 an dem Gesicht des Patienten ergibt. Vorzugsweise sind eine erste und eine zweite Befestigungslasche 11, 12 einem jeweiligen Nasenflügelabschnitt 4, 5 des Nasenaufnahmeraumes 2 zugeordnet und insbesondere an diesen angeordnet. Beispielsweise sind die Befestigungslaschen 11, 12 an einer Außenfläche der Wandung 3 an dem entsprechenden Nasenflügelabschnitt 4, 5 angeordnet. Beispielsweise ist die erste Befestigungslasche 11 einem ersten Nasenflügelabschnitt 4 und die zweite Befestigungslasche 12 einem zweiten Nasenflügelabschnitt 5 zugeordnet. Die erste Befestigungslasche 11 und die zweite Befestigungslasche 12, insbesondere die jeweiligen Durchbrüche 14, sind beispielsweise auf einer gemeinsamen ersten Befestigungsachse 15 der Beatmungsmaske 1 angeordnet. Eine dritte Befestigungslasche 13 ist vorzugsweise dem Nasenwurzelabschnitt 6 des Nasenaufnahmeraumes 2 zugeordnet und insbesondere an diesem angeordnet. Beispielsweise ist die Befestigungslasche 13 an der Außenfläche der Wandung 3 an dem Nasenwurzelabschnitt 6 angeordnet. Die dritte Befestigungslasche 13 bzw. der Durchbruch 14 der dritten Befestigungslasche 13 ist vorzugsweise auf einer senkrecht zu der ersten Befestigungsachse 15 ausgebildeten zweiten Befestigungsachse 16 angeordnet. Die Befestigungslaschen 11, 12, 13 bzw. die jeweiligen Durchbrüche 14 der Befestigungslaschen 11, 12, 13 bilden so vorzugsweise im Wesentlichen die Ecken eines gleichschenkeligen oder gleichseitigen Dreieckes. Die Befestigungslaschen 11, 12, 13 erstrecken sich von dem Nasenaufnahmeraum 2 weg in die Umgebung der Beatmungsmaske 1 hinein.

Die Auflagefläche 17 der Beatmungsmaske 1 ist vorzugsweise als umlaufender Saum 17 ausgebildet, welcher den Nasenaufnahmeraum 2 zu dem Gesicht des Patienten hin zumindest teilweise abschließt. Der umlaufende Saum 17 dient zur flächigen Auflage der Beatmungsmaske 1 auf dem Gesicht des Patienten. Der umlaufende Saum 17 schließt den Nasenaufnahmeraum 2 vorzugsweise zu dem Gesicht des Patienten hin bis auf einen in etwa T-förmigen Durchbruch 18 hin ab. Der Saum 17 weist beispielsweise Dichtlappen 19, 20 auf, welche flexibel sind und an den Nasenflügeln der Nase des Patienten aufliegen. Der Saum 17 ist vorzugsweise einstückig mit der Wandung 3 des Nasenaufnahmeraumes 2 ausgebildet. Der Saum 17 ermöglicht eine Abdichtung der Beatmungsmaske 1 gegen das Gesicht des Patienten und erhöht aufgrund der vergrößerten Auflagefläche den Tragekomfort für den Patienten. Die Befestigungslaschen 11, 12, 13 können an dem Saum 17 vorgesehen sein.

Die Beatmungsmaske 1 weist ferner eine in dem umlaufenden Saum 17 vorgesehene umlaufende Nut 21 auf. Die Nut 21 ist beispielsweise als halbkreisförmige Vertiefung oder Einwölbung in dem Saum 17 vorgesehen. Die Nut 17 dient vorzugsweise zur zumindest abschnittsweisen Aufnahme eines Dichtmittels 22, insbesondere eines Silikongels 22. Das Dichtmittel 22 dient der Abdichtung der Beatmungsmaske 1 gegen das Gesicht des Patienten. Zum Schutz des Dichtmittels 22 vor dem Gebrauch der Beatmungsmaske 1 ist dieses beispielsweise mit einer abziehbaren Schutzfolie 23 abgedeckt. Die Schutzfolie 23 ist beispielsweise als Polyethylenfolie ausgebildet. Vorzugsweise schließt die Schutzfolie 23 auch den Nasenaufnahmeraum 2 vor dem Gebrauch der Beatmungsmaske 1 vollständig ab. Hierdurch ist der Nasenaufnahmeraum 2 vor dem Gebrauch der Beatmungsmaske 1 vor Verschmutzung geschützt. Die Beatmungsmaske 1 mit dem Dichtmittel 22 und der Schutzfolie 23 bildet beispielsweise eine Verpackungseinheit 42 oder Verpackungsanordnung 42, welche als Zubehör für eine Beatmungsvorrichtung erhältlich ist.

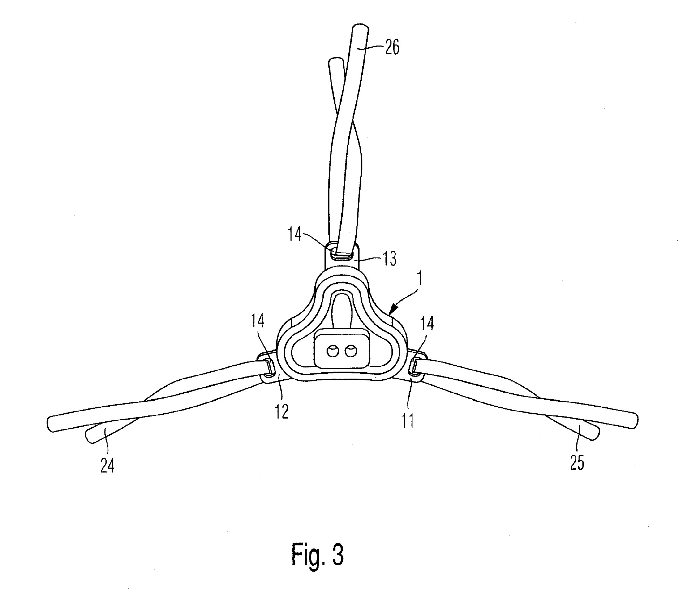

Die Fig. 3 illustriert die Beatmungsmaske 1 gemäß der Fig. 1 und 2 in einer Vorderansicht. Befestigungsmittel 24, 25, 26 zur Befestigung der Beatmungsmaske 1 an dem Patienten sind durch die jeweiligen Durchbrüche 14 der Befestigungslaschen 11, 12, 13 geführt. Die Befestigungsmittel 24, 25, 26 sind vorzugsweise als Klett- oder Velcrobänder 24, 25, 26 ausgebildet. Alternativ können die Befestigungsmittel 24, 25, 26 auch Druckknopfverbindungen oder beliebige andere wieder lösbare Verbindungen aufweisen. Die Klettbänder 24, 25, 26 sind vorzugsweise derart ausgebildet, dass jedes Klettband 24, 25, 25 zumindest einen Flauschabschnitt oder Schlingenabschnitt und zumindest einen Hakenabschnitt zum Eingreifen in einen entsprechenden Flauschabschnitt oder Schlingenabschnitt aufweist. Der entsprechende Flauschabschnitt oder Schlingenabschnitt kann an demselben Klettband 24, 25, 25 oder an einer anderen Komponente, beispielsweise einem Kleidungsstück, vorgesehen sein. Die Klettbänder 24, 25, 26 können einseitig oder beidseitig mit Hakenabschnitten und/oder Flauschabschnitten oder Schlingenabschnitten versehen sein.

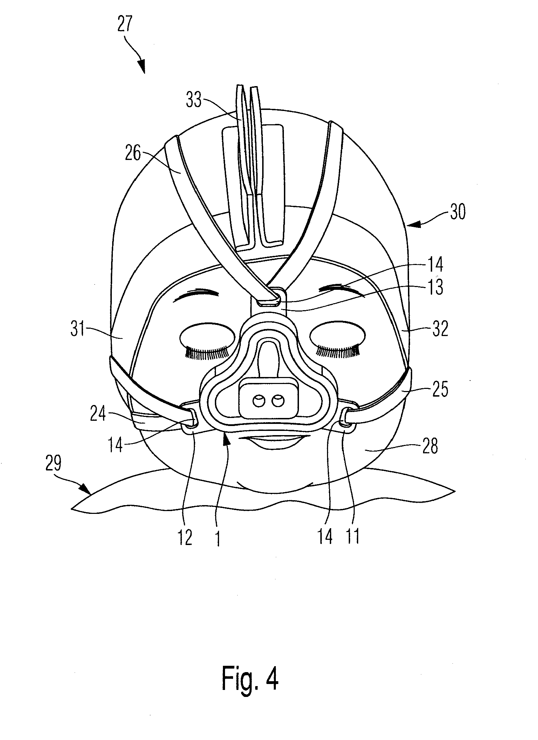

Die Fig. 4 illustriert eine Beatmungsmaskenanordnung 27 mit einer Beatmungsmaske 1 nach den Fig. 1 bis 3 und mit einer auf dem Kopf 28 des Patienten 29 angeordneten Mütze 30. Die Mütze 30 kann als Einmalartikel oder als wieder verwendbarer, insbesondere sterilisierbarer, Artikel ausgebildet sein. Vorzugsweise wird die Mütze 30 in verschiedenen Größen und Formen zur Verfügung gestellt, um eine möglichst gute Anpassung an den Kopf 28 des Patienten 29 zu erreichen.

Die Befestigungsmittel 24, 25, 26 sind zum Fixieren der Beatmungsmaske 1 an dem Kopf 28 des Patienten 29 sowohl mit den Befestigungslaschen 11, 12, 13 der Beatmungsmaske 1 als auch mit der Mütze 30 in Wirkverbindung. Die Befestigungsmittel 24, 25, 26 sind durch die jeweiligen Durchbrüche 14 der Befestigungslaschen 11, 12, 13 geführt. Die Befestigungsmittel 24, 25, 26 stehen vorzugsweise mit dem Gewebe der Mütze 30 in Wirkeingriff. Beispielsweise sind Abschnitte der Mütze 30 oder die gesamte Mütze 30 mit einem Material gebildet, welches beispielsweise mit Hakenabschnitten der Befestigungsmittel 24, 25, 26 in Wirkeingriff bringbar ist. Beispielsweise ist die Mütze 30 mit einem zu den Hakenabschnitten der Befestigungsmittel 24, 25, 26 komplementären Flauschmaterial oder Schlingenmaterial gebildet. Hierdurch ist die Beatmungsmaske 1 sicher an dem Kopf 28 des Patienten 29 fixiert. Vorzugsweise ist jeweils ein Befestigungsmittel 24, 25 mit einem jeweiligen Ohrabschnitt 31, 32 und mit einer jeweiligen Befestigungslasche 11, 12 in Wirkverbindung. Das Befestigungsmittel 26 ist vorzugsweise mit einem Stirnabschnitt 33 und mit der dem Nasenwurzelabschnitt 6 der Beatmungsmaske 1 zugeordneten Befestigungslasche 13 in Wirkverbindung. Hierdurch ist auch ein sicherer Sitz der Beatmungsmaske 1 auf der Nasenwurzel der Nase des Patienten 29 gewährleistet, wodurch sich die Zuverlässigkeit der Beatmungsmaske erhöht.

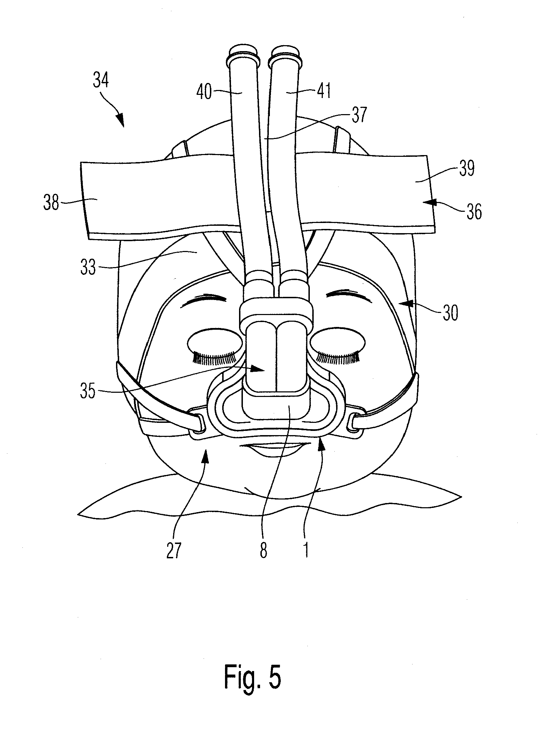

Die Fig. 5 illustriert ein Beatmungssystem 34 mit einer Beatmungsmaskenanordnung 27 gemäß der Fig. 4. Das Beatmungssystem 34 weist ferner eine Beatmungsvorrichtung 35 zum Erzeugen eines kontinuierlichen positiven Atemwegdrucks auf. Die Beatmungsvorrichtung 35 ist beispielsweise über Anschlussschläuche mit einer nicht dargestellten Beatmungspumpe verbunden. Die Beatmungsvorrichtung 35 steht mit dem Anschlussstück 8 der Beatmungsmaske 1 in Wirkverbindung. Eine derartige Beatmungsvorrichtung 35 ist beispielsweise in der deutschen Gebrauchsmusterschrift DE 202 06 695 U1 beschrieben.

Die Mütze 30 weist vorzugsweise eine an ihrem Stirnabschnitt 33 angeordnete Befestigungseinrichtung 36 zur Befestigung der Beatmungsvorrichtung 35 an der Mütze 30 auf. Die Befestigungseinrichtung 36 ist vorzugsweise als Kletteinrichtung 36 ausgebildet. Alternativ kann die Befestigungseinrichtung 36 auch eine Druckknopfverbindung, Schnürverbindung, Hakenverbindung oder eine beliebige andere wieder lösbare Verbindung aufweisen. Die Kletteinrichtung 35 weist beispielsweise ein zentrales , beidseitiges Flausch- oder Schlingenband 37 und zwei beidseitig des Flauschbandes 37 angeordnete Hakenbänder 38, 39 auf. Die Befestigungseinrichtung 36 ist beispielsweise mit dem Stirnabschnitt 33 der Mütze 30 vernäht oder verklebt. Die Befestigungseinrichtung 36 kann auch als von der Mütze 30 ablösbar ausgebildet sein.

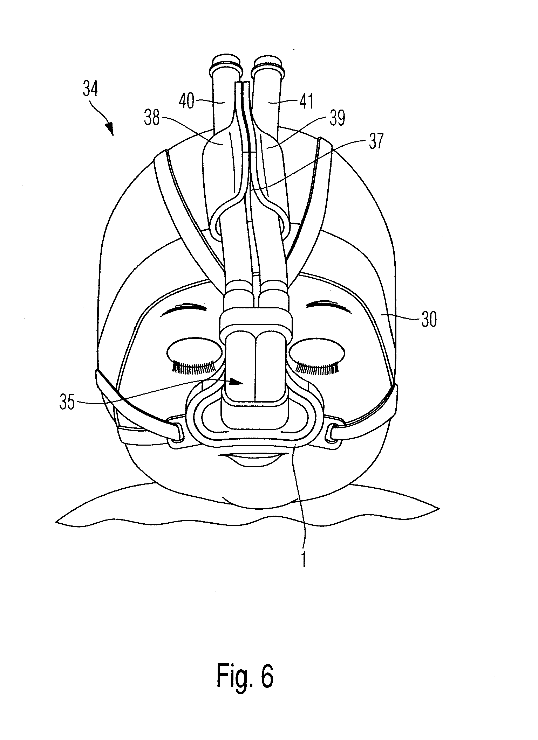

Die Fig. 5 stellt die Befestigungseinrichtung 36 in einem geöffneten Zustand dar. Zur Befestigung der Beatmungsvorrichtung 35 an der Mütze 30 wird beispielsweise das zentrale Flauschband 37 der Befestigungseinrichtung 36 zwischen zwei Anschlussschläuchen 40, 41 der Beatmungsvorrichtung 35 hindurchgeführt und die jeweiligen Hakenbänder 38, 39 werden um die Anschlussschläuche 40, 41 herumgeführt und mit dem Flauschband 37 in Wirkverbindung gebracht. Hierdurch ist die Beatmungsvorrichtung 35, wie in Fig. 6 angedeutet, sicher an dem Kopf 28 des Patienten 29 fixiert.

### Bezugszeichenliste

- 1: Beatmungsmaske
- 2: Nasenaufnahmeraum
- 3: Wandung
- 4: Nasenflügelabschnitt
- 5: Nasenflügelabschnitt
- 6: Nasenwurzelabschnitt
- 7: Nasenspitzenabschnitt
- 8: Anschlussstück
- 9: Durchbruch
- 10: Durchbruch
- 11: Befestigungslasche
- 12: Befestigungslasche
- 13: Befestigungslasche
- 14: Durchbruch
- 15: Befestigungsachse
- 16: Befestigungsachse
- 17: Auflagefläche
- 18: Durchbruch
- 19: Dichtlappen
- 20: Dichtlappen
- 21: Nut
- 22: Dichtmittel
- 23: Schutzfolie
- 24: Befestigungsmittel
- 25: Befestigungsmittel
- 26: Befestigungsmittel
- 27: Beatmungsmaskenanordnung
- 28: Kopf
- 29: Patient
- 30: Mütze
- 31: Ohrabschnitt
- 32: Ohrabschnitt
- 33: Stirnabschnitt
- 34: Beatmungssystem
- 35: Beatmungsvorrichtung
- 36: Befestigungseinrichtung
- 37: Flauschband
- 38: Hakenband
- 39: Hakenband
- 40: Anschlussschlauch
- 41: Anschlussschlauch
- 42: Verpackungseinheit

## Patentansprüche

1. Beatmungsmaske (1) zum künstlichen Beatmen eines Patienten (29), mit:
einem Nasenaufnahmeraum (2); und
mindestens drei Befestigungslaschen (11, 12, 13) zum Befestigen der Beatmungsmaske (1) an dem Patienten (29), wobei jeweils eine erste und zweite Befestigungslasche (11, 12) an einem jeweiligen Nasenflügelabschnitt (4, 5) des Nasenaufnahmeraumes (2) und eine dritte Befestigungslasche (13) an einem Nasenwurzelabschnitt (6) des Nasenaufnahmeraumes (2) angeordnet ist.

2. Beatmungsmaske nach Anspruch 1, **dadurch gekennzeichnet, dass** die erste Befestigungslasche (11) und die zweite Befestigungslasche (12) auf einer gemeinsamen ersten Befestigungsachse (15) angeordnet sind und dass die dritte Befestigungslasche (13) auf einer senkrecht zu der ersten Befestigungsachse (15) ausgebildeten zweiten Befestigungsachse (16) angeordnet ist.

3. Beatmungsmaske nach Anspruch 1 oder 2, **dadurch gekennzeichnet,**
**dass** die Beatmungsmaske (1) einen umlaufenden Saum (17) zur Auflage auf dem Gesicht des Patienten (29) aufweist.

4. Beatmungsmaske nach Anspruch 3, **dadurch gekennzeichnet, dass** der Saum (17) eine umlaufende Nut (21) zur Aufnahme eines Dichtmittels (22), insbesondere eines Silikongels (22), aufweist.

5. Beatmungsmaske nach Anspruch 3 oder 4, **dadurch gekennzeichnet,**
**dass** die Befestigungslaschen (11, 12, 13) an dem Saum (17) angeordnet sind.

6. Beatmungsmaskenanordnung (27), mit:
einer Beatmungsmaske (1) nach einem der vorhergehenden Ansprüche;
einer auf dem Kopf (28) des Patienten (29) angeordneten Mütze (30); und
Befestigungsmitteln (24, 25, 26), welche zum Fixieren der Beatmungsmaske (1) an dem Kopf (28) des Patienten (29) mit den Befestigungslaschen (11, 12, 13) und mit der Mütze (30) in Wirkverbindung sind.

7. Beatmungsmaskenanordnung nach Anspruch 6, **dadurch gekennzeichnet,**
**dass** die Befestigungsmittel (24, 25, 26) als Klettbänder (24, 25, 26) ausgebildet sind.

8. Beatmungsmaskenanordnung nach Anspruch 6 oder 7, **dadurch gekennzeichnet,**
**dass** jeweils ein Befestigungsmittel (24, 25) mit einem jeweiligen Ohrabschnitt (31, 32) der Mütze (30) und ein Befestigungsmittel (26) mit einem Stirnabschnitt (33) der Mütze (30) in Wirkverbindung ist.

9. Beatmungssystem (34), mit:
einer Beatmungsmaskenanordnung (27) nach einem der Ansprüche 6 bis 8; und
einer Beatmungsvorrichtung (35) zum Erzeugen eines kontinuierlichen positiven Atemwegdrucks.

10. Beatmungssystem nach Anspruch 9, **dadurch gekennzeichnet, dass** die Mütze (30) an einem Stirnabschnitt (33) eine Befestigungseinrichtung (36) zur Befestigung der Beatmungsvorrichtung (35) an der Mütze (30) aufweist.

11. Beatmungssystem nach Anspruch 10, **dadurch gekennzeichnet, dass** die Befestigungseinrichtung (36) als Kletteinrichtung (36) ausgebildet ist.

12. Verpackungseinheit (42), mit:
einer Beatmungsmaske (1) nach einem der Ansprüche 1 bis 5, wobei die Beatmungsmaske (1) einen Saum (17) mit einer umlaufenden Nut (21) aufweist;
ein Dichtmittel (22), welches zur Abdichtung der Beatmungsmaske (1) gegen das Gesicht des Patienten (29) zumindest abschnittsweise in der umlaufenden Nut (21) angeordnet ist; und
einer Schutzfolie (23) zum Abdecken des Dichtmittels (22) bis zum Gebrauch der Beatmungsmaske (1).

13. Verpackungseinheit nach Anspruch 12,
**dadurch gekennzeichnet,**
**dass** das Dichtmittel (22) als Silikongel (22) ausgebildet ist.
